# EUROPEAN PATENT APPLICATION

(11) **EP 4 389 078 A1**
(43) Date of publication of application: **26.06.2024**
(21) Application number: 23218191.7
(22) Date of filing: 19.12.2023
(51) Int. Cl.: A61F 2/30, A61F 2/38

(54) **SEGMENTED KEEL FOR PROSTHETIC IMPLANT HAVING A STEM**

(30) Priority: 22.12.2022 US 202263434566 P; 01.12.2023 US 202318526955
(71) Applicant: Zimmer, Inc., Warsaw, IN 46580 (US)
(72) Inventor: YOKO, Tim, Granger, 46530 (US); BARKER, Joshua, Warsaw, 46582 (US)
(74) Representative: Taor, Simon Edward William

(57) **Abstract**

A tibial implant comprises a tibial tray component comprising a tray plate with proximal and distal surfaces, a stem and a segmented keel extending from the distal surface, the segmented keel including a first wing having a first notch proximate the stem, and a first taper extending along the stem in a first axial position axially aligned with the segmented keel. A sleeve for a stem of a prosthetic implant comprises an annular body, an inner passage extending through the annular body, the inner passage comprising a Morse taper, and a first slot extending into the annular body to intersect the inner passage and the Morse taper. A method comprises fabricating a stemmed prosthetic component from a metallic material, advancing a machining tool into a notch in a keel wing extending from the stem, and forming a Morse taper on the stem with the machining tool in the notch.

## Description

### CLAIM OF PRIORITY

This application claims the benefit of U.S. Provisional Patent Application Serial No. 63/434,566, filed on December 22, 2022, and U.S. Patent Application Serial No. 18/526,955, filed on December 1, 2023 the benefit of priority of which is claimed hereby, and which is incorporated by reference herein in its entirety.

### TECHNICAL FIELD

The present disclosure pertains generally, but not by way of limitation, to prosthetic implant devices having stems configured to be inserted into bone. More specifically, but not by way of limitation, the present application relates to prosthetic tibial and femoral components having keeled stems for engaging with bone matter.

### BACKGROUND

Prosthetic implant devices, such as femoral and tibial components, sometimes include a stem extending from an articulating component. For example, a tibial component can include a tray that attaches to a bearing component and that seats against a resected surface of a tibia at the epiphysis portion of the bone. A stem can extend from the tray into the tibia along a length of the diaphysis portion of the bone. Sometimes the metaphysis portion of the tibia below the epiphysis portion can include damaged or unhealthy cancellous bone at the resection. As such, it is sometimes desirable to remove weakened bone material, such as cancellous bone, with a broach or reamer to leave a space in the metaphyseal portion of the bone larger than the stem. Sometimes a sleeve is mechanically coupled to the stem of a tibial component in order to fill the space and provide engagement with cortical bone or other bone matter. Additionally, a cone can be placed around, but spaced from, the stem to engage bone matter. Prosthetic implant devices can thus be part of a system of components whereby different accessories, including sleeves, cones and keels, can be attached to the stem, which allows a surgeon the ability to select an accessory that provides the best outcome for the patient.

Examples of attachable accessories for prosthetic implants having stems are described in U.S. Pat. No. 8,721,733 to Bonitati; U.S. Pat. No. 10,835,382 to Habegger et al.; U.S. Pat. No. 10,987,225 to Yoko et al.; U.S. Pub. No. 2014/0277528 to Mines et al.; U.S. Pub. No. 2014/0277540 to Leszko et al.; and U.S. Pub. No. 2017/0000503 to Keefer et al.

### OVERVIEW

The present inventors have recognized that the use of a linked total knee prosthesis where the tibial component and femoral component are connected via a linkage can result in additional torque being applied to the tibial component from the femoral component. Such additional torque can result in disruption between the prosthetic tibial component and bone matter. As such, the present inventors have recognized the desirability of providing additional anchoring features or anti-rotation features between the tibial component and bone matter.

The present subject matter can provide solutions to this problem and other problems, such as by providing a prosthetic implant component having a stem with an integrated keel. The integrated keel can provide resistance to rotation of the tibial component from various sources, such as movement of the joint and the femoral component. As such, the present subject matter can facilitate the use of a linked total knee prosthesis that can provide more stable and realistic performing total knee constructs.

The present inventors have recognized, among other things, that problems to be solved in the use of stemmed implants can include the undesirability of having to assemble components of a prosthetic implant during a surgical procedure. As mentioned, depending on the bone matter of a specific patient, it can be advantageous to position an accessory, such as a sleeve, cone or keel, around a stem of a tibial component. However, in some cases, the stem can be used without any accessory at all. As such, a typical surgical procedure can involve having to attach an accessory component, such as a sleeve or keel, to the main prosthetic component, leaving other accessory components unused. Furthermore, using none or one of multiple accessories results in unused accessories that must be reprocessed before reuse or disposed of without ever being used.

The present subject matter can provide solutions to this problem and other problems, such as by providing a prosthetic implant component having a stem with an integrated keel having segmented wings. The segmented wings can be configured to receive a sleeve, such as by including slots or notches. The segmented wings can function as an integrated keel without the use of an accessory. Furthermore, slots within the segmented wings can allow for a sleeve to be brought into engagement with the stem closer to the tibial tray and to facilitate interlocking along a greater length of the stem. The segmented keel can still allow for the use of a cone. As such, tibial components having integrated keels with segmented wings can thereby reduce the number of accessory components, particularly a separate keel component, and can eliminate the need to perform assembly of an accessory with the stem when functionality of a keel is desired. As such, a tibial component set or system can include only the tibial tray component with an integrated and segmented keel, a cone and a sleeve. Such a system eliminates the need to manufacture and assemble a separate keel accessory.

The present inventors have also recognized that it can be difficult to couple accessory components to prosthetic implants having stems. In particular, it can be difficult to attach accessory components to stems having a keel in place due to potential interference with a tapered connection, such as a Morse taper. A Morse taper involves mating carefully machined outer and inner surfaces of the stem and the sleeve. However, the presence of wings of a keel at the stem can interfere with the ability of an inner surface of a sleeve to abut the outer surface of the stem.

The present subject matter can provide solutions to this problem and other problems, such as by providing a keel having segmented wings that can allow a machining tool to advance along the outer surface of a stem to produce a conical taper surface without interference from wings of a keel. As such, the machining tool can be brought closer to the tibial tray to form the tapered surface closer to the tibial tray. Thereafter, an accessory component, such as a sleeve, can have a longer length of engagement for a tapered connection in close proximity to the tray.

In an example, a tibial implant can comprise a tibial tray component comprising a tray plate comprising a proximal surface and a distal surface, a stem extending from the distal surface, a segmented keel extending from the distal surface, wherein the segmented keel includes a first wing having a first notch proximate the stem, and a first taper extending along the stem in a first axial position axially aligned with the segmented keel.

In another example, a sleeve for a stem of a prosthetic implant can comprise an annular body, an inner passage extending through the annular body, wherein the inner passage comprises a Morse taper, and a first slot extending into the annular body to intersect the inner passage and the Morse taper.

In another example, a prosthetic implant can comprise a prosthetic component comprising an articulating component, a stem extending from the articulating component, and a first segmented keel wing extending form the stem, and a slotted sleeve comprising an annular body, an inner passage extending through the annular body configured to receive the stem, and a first slot extending into the annular body to intersect the inner passage, the first slot configured to align with the first segmented keel wing.

In an additional example, a method of manufacturing a stemmed prosthetic component can comprise fabricating the stemmed prosthetic component from a metallic material, positioning a machining tool around a stem extending from the stemmed prosthetic component, advancing the machining tool into a notch in a keel wing extending from the stem, and forming a Morse taper on the stem with the machining tool in the notch.

This overview is intended to provide an overview of subject matter of the present patent application. It is not intended to provide an exclusive or exhaustive explanation of the invention. The detailed description is included to provide further information about the present patent application.

### BRIEF DESCRIPTION OF THE DRAWINGS

FIG. 1A is a front, or anterior, perspective view of a prosthetic knee assembly comprising a femoral component configured for linked rotation with a tibial component.
FIG. 1B is an exploded view of the prosthetic knee assembly of FIG. 1A showing various components including a segmented keel configured for use with a slotted sleeve.
FIG. 2 is a cross-sectional view of the prosthetic knee assembly of FIG. 1A showing a shackle connecting a hinge axle of the femoral component and a hinge post of the tibial component.
FIG. 3A and FIG. 3B are top and bottom exploded views of the tibial component of FIGS. 1A - 2 showing a tibial tray, slotted sleeve, bushing and cap.
FIG. 4A and FIG. 4B are top and bottom, or superior and inferior, perspective views of the tibial component of FIGS. 3A and 3B without the tibial bearing component and with a slotted sleeve attached to the segmented keel.
FIG. 5 is a side cross-sectional view of the tibial component of FIGS. 4A and 4B showing the slotted sleeve seated against a stem of the tibial component.
FIG. 6A is a rear, or posterior, view of the tibial tray of FIGS. 1-5 showing a pair of segmented keel wings extending from the stem.
FIG. 6B is a close-up view of the tibial tray of FIG. 6A showing cut-outs forming the segmented keel wings at the stem.
FIG. 7A is a front perspective view of the slotted sleeve of FIGS. 1-5 showing slots extending from a central passage.
FIG. 7B is a bottom perspective view of the slotted sleeve of FIG. 7A showing slots extending through to an exterior surface.
FIG. 7C is a front view of the slotted sleeve of FIGS. 7A and 7B showing side lobes extending from a main body.
FIG. 7D is a top view of the slotted sleeve of FIGS. 7A - 7C showing internal ledges along the central passage.
FIG. 8 is cross-sectional view of the tibial tray of FIGS. 6A and 6B assembled with the slotted sleeve of FIGS. 7A - 7D taken along a segmented keel wing to show engagement of the slotted sleeve with the stem on anterior and posterior sides of the stem.
FIG. 9A, FIG. 9B, FIG. 9C and FIG. 9D are cross-sectional views of the slotted sleeve assembled onto the tibial tray showing engagement between the segmented keel and the slotted sleeve at various positions along the stem.
FIG. 10 is a bottom rear perspective view of the tibial tray of FIGS. 6A and 6B assembled with a winged sleeve.
FIG. 11 is a top front perspective view of the winged sleeve of FIG. 10 showing wings of the sleeve configured to fill-in slots in wings of the keel.

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

### DETAILED DESCRIPTION

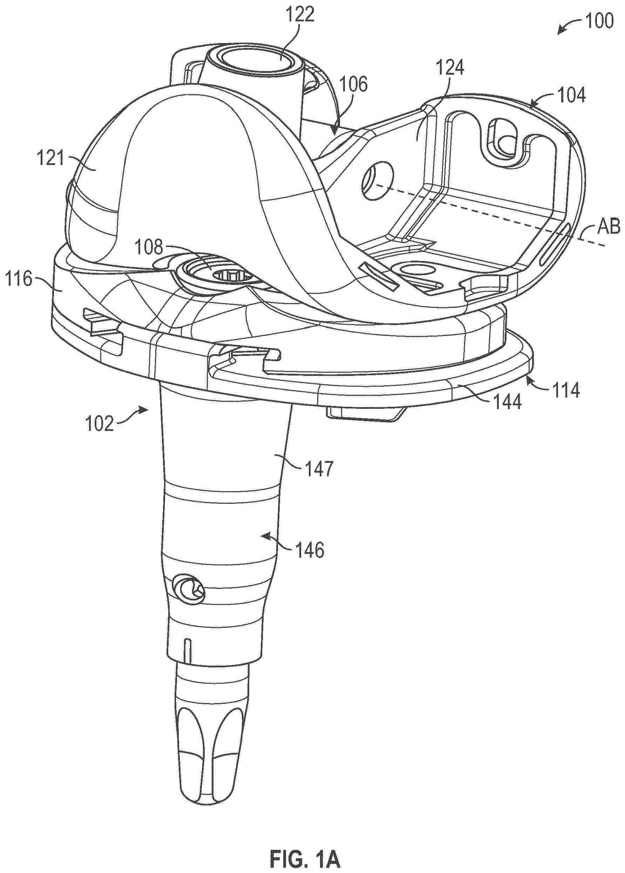

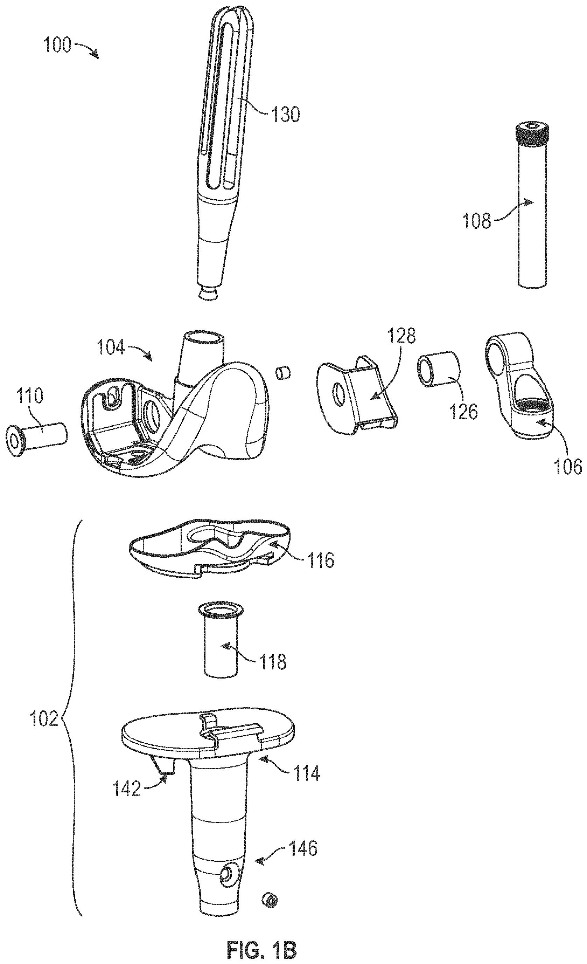

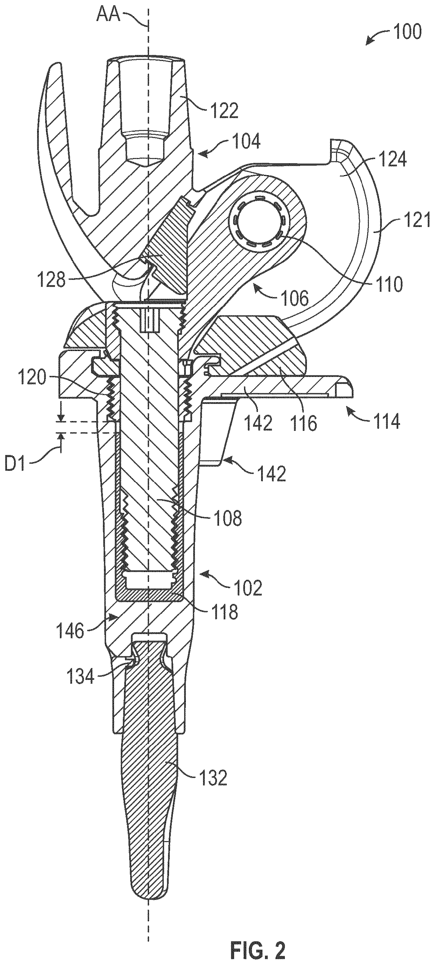

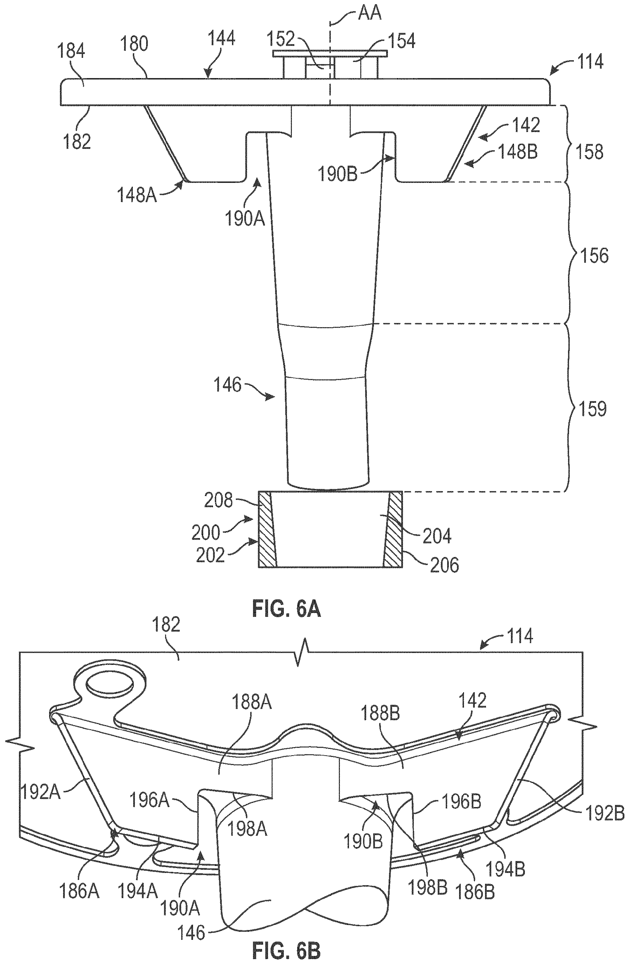

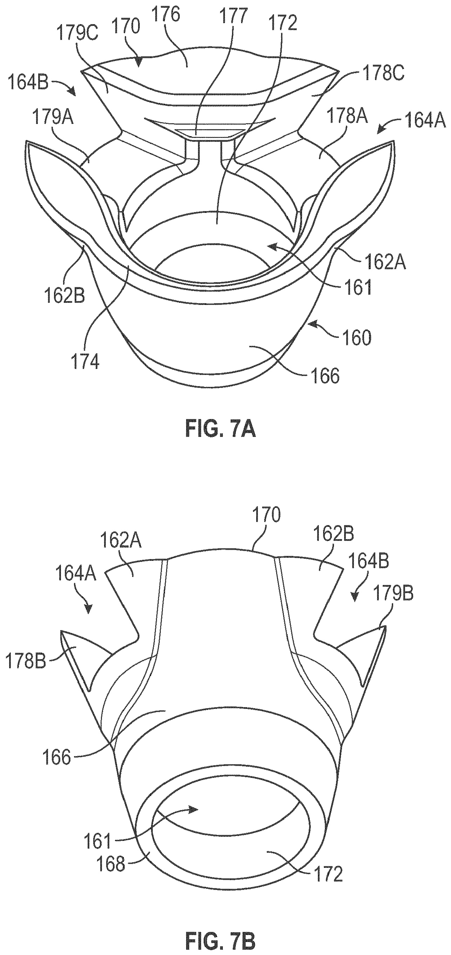

FIG. 1A is a front perspective view of prosthetic knee assembly 100 comprising tibial component 102 configured for linked rotation with femoral component 104. FIG. 1B is an exploded view of prosthetic knee assembly 100 of FIG. 1A showing various components including segmented keel 142 (FIGS. 6A and 6B) configured for use with slotted sleeve 140 (FIGS. 7A - 7D). FIG. 2 is a cross-sectional view of prosthetic knee assembly 100 of FIG. 1A showing shackle 106 connecting hinge post 108 of tibial component 102 and hinge axle 110 of femoral component 104. FIGS. 1A- 2 are discussed concurrently.

Tibial component 102 can comprise tibial tray 114, bearing component 116, bushing 118 and cap 120. Femoral component 104 can comprise bearing surfaces 121, stem socket 122 and sidewalls 124. Additionally, femoral component 104 can be connected to extension 130 (FIG. 1B) as stem socket 122 and tibial component 102 can be connected to extension 132 (FIG. 2) at socket 134 (FIG. 2).

Tibial component 102 and femoral component 104 can be connected using shackle 106, hinge post 108 and hinge axle 110. Shackle 106 can rotate about hinge post 108 along axis AA to allow femoral component 104 to rotate relative to tibial component 102. Shackle 106 can pivot about hinge axle 110 at axis AB to allow femoral component 104 to roll along tibial component 102. As such, bearing surfaces 121 can roll along bearing component 116 in the anterior-posterior direction and can twist against bearing component 116 along a superior-inferior axis.

Shackle 106 can mechanically link femoral component 104 and tibial component 102 by hinge post 108 attaching shackle 106 to tibial tray 114 and hinge axle 110 attaching shackle 106 to sidewalls 124. A portion of shackle 106 can be received in a recess in bearing component 116 and this portion can be threaded or otherwise connected to hinge post 108. Hinge post 108 can extend distally and can be received in a recess of tibial component 102 and the recess of bearing component 116. Hinge post 108 can be moveable (e.g., rotatable and/or capable of distraction) relative to one or more of femoral component 104 or tibial tray 114. For example, hinge post 108 can be rotatably connected to femoral component 104 via hinge axle 110, which can cause movement of femoral component about axis AA, as explained in greater detail below.

When assembled, shackle 106 can be placed between opposing sidewalls 124 of femoral component 104. Poly box 128 can be positioned between sidewalls 124 and shackle 106. Additionally, axle bushing 126 can be positioned over hinge axle 110 within an aperture on a proximal portion of shackle 106. Shackle 106 and hinge post 108 can be formed from suitable materials such as a titanium alloy, a cobalt-chromium alloy, etc., while axle bushing 126 and poly box 128 can be formed from different materials, such as plastic, e.g., UHMWPE. Axle bushing 126 can act as a bearing between shackle 106 and hinge axle 110. Poly box 128 can act as a bearing between femoral component 104 and shackle 106.

Connected as such, femoral component 104 and tibial component 102 can operate in conjunction with each other to replace natural knee ligaments, such as the anterior cruciate ligament (ACL) and posterior cruciate ligament (PCL), to replicate the natural motion and feel of an anatomic joint. As mentioned above, the linking of femoral component 104 to tibial component 102 can transfer additional loading to tibial component 102, thereby making it desirable to provide additional anchoring to tibial component 102. As discussed herein, tibial component 102 can include segmented keel 142 to provide anti-rotational engagement with bone. Furthermore, segmented keel 142 can engage with a sleeve along a tapered interface in close proximity to tray plate 144 despite the presence of segmented keel 142 due to the segmented keel wings described herein.

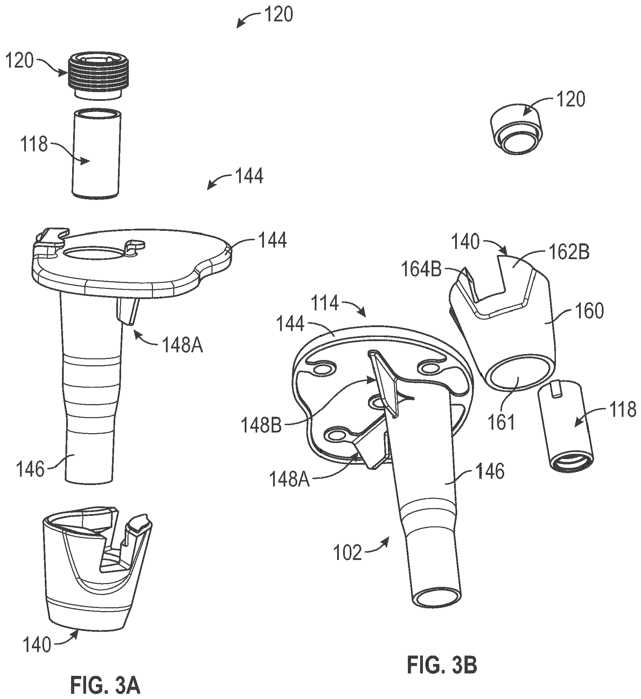

FIG. 3A is top exploded view of tibial component 102 of FIGS. 1A - 2 showing tibial tray 114, slotted sleeve 140, bushing 118 and cap 120. FIG. 3B is a bottom exploded view of tibial component 102 of FIGS. 1A - 2. FIGS. 3A and 3B are discussed concurrently.

Bushing 118 can be inserted into bore 150 and can comprise a sleeve into which hinge post 108 can be inserted. For example, bushing 118 can be force fit or loosely fit into bore 150 and can include internal threads to receiving mating external threads on hinge post 108. Cap 120 can be threaded into mating threads on bore 150 to prevent egress of bushing 118 out of bore 150 in the proximal direction. In examples, the top or superior surface of bushing 118 can be spaced from the bottom or inferior surface of cap 120 a distance D1 (FIG. 2) to provide anti-luxation travel. For example, the gap provided by distance D1 can allow femoral component 104 to move proximally relative to tibial component 102 to prevent luxation of the patella or to accommodate rolling of bearing surfaces 121 of femoral component 104 against bearing component 116 of tibial component 102. In examples, distanceD 1 can be approximately 8 mm (~0.315 inches). However, in additional examples of the present disclosure, cap 120 and bushing 118 can be integrated into a single component to prevent superior movement of femoral component 104 relative to tibial component 102.

Due to linkage of femoral component 104 (FIG. 1A) to tibial component 102, tibial tray 114 can be subject to additional rotational forces in a transverse plane along superior-inferior axis AA. In order to counteract rotational movement about axis AA, tibial tray 114 can include segmented keel 142 to provide anchoring in bone matter in directions extending radially from axis AA. If desired, a surgeon can position slotted sleeve 140 onto stem 146 to occupy additional space within a bone to provide additional anchoring. For example, segmented keel 142 can be used in cancellous bone within the proximal portion of the resected tibia engaging tray plate 144 when the cancellous bone is generally healthy or otherwise has adequate density to receive wings 148A and 148B of segmented keel 142 to provide rotational resistance. If, however, the cancellous bone that would surround stem 146 is unhealthy or otherwise not sufficiently dense, slotted sleeve 140 can be fit around and attached to stem 146 to displace (either with or without removal via reaming or broaching) inadequate cancellous bone and engage healthy or dense bone matter including, in some cases, engagement with cortical bone.

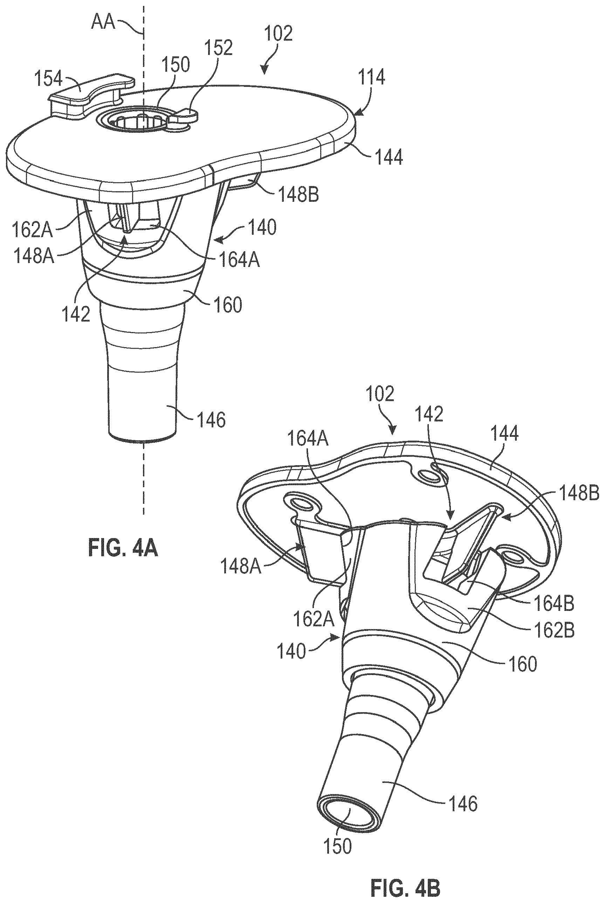

FIG. 4A is a top perspective view of tibial component 102 of FIGS. 3A and 3B without bearing component 116 and with slotted sleeve 140 attached to segmented keel 142. FIG. 4B is a bottom perspective view of tibial component 102 of FIG. 4A showing slotted sleeve 140 attached to segmented keel 142. FIGS. 4A and 4B are discussed concurrently.

Tibial tray 114 can comprise tray plate 144 and stem 146. Segmented keel 142 can be connected to tray plate 144 and stem 146. Tibial tray 114 can additionally comprise bore 150 extending into tray plate 144 and stem 146 and features for attaching bearing component 116, such as hook 152 and ridge 154. Slotted sleeve 140 can comprise body 160, central passage 161, lobe 162A, lobe 162B, slot 164A and slot 164B.

Bore 150 can allow for the passage of bushing 118 (FIG. 1B) and hinge post 108 (FIG. 1B) into stem 146. Hook 152 and ridge 154 can be configured to attach bearing component 116 bearing component 116 to tray plate 144.

Segmented keel 142 can comprise wings 148A and 148B that can extend radially outward from stem 146 and axially from tray plate 144 in an inferior direction. Thus, wings 148A and 148B can extend into bone matter, such as cancellous bone, against which tray plate 144 engages. As discussed in greater detail with reference to FIGS. 6A and 6B, wings 148A and 148B can comprise slot 190A and slot 190B, respectively, to allow for machining of stem 146 and to allow for slotted sleeve 140 to be brought into engagement with stem 146. As discussed in greater detail with reference to FIGS. 7A - 7D, slotted sleeve 140 can include slot 164A and slot 164B to allow slotted sleeve 140 to fit around wings 148A and 148B.

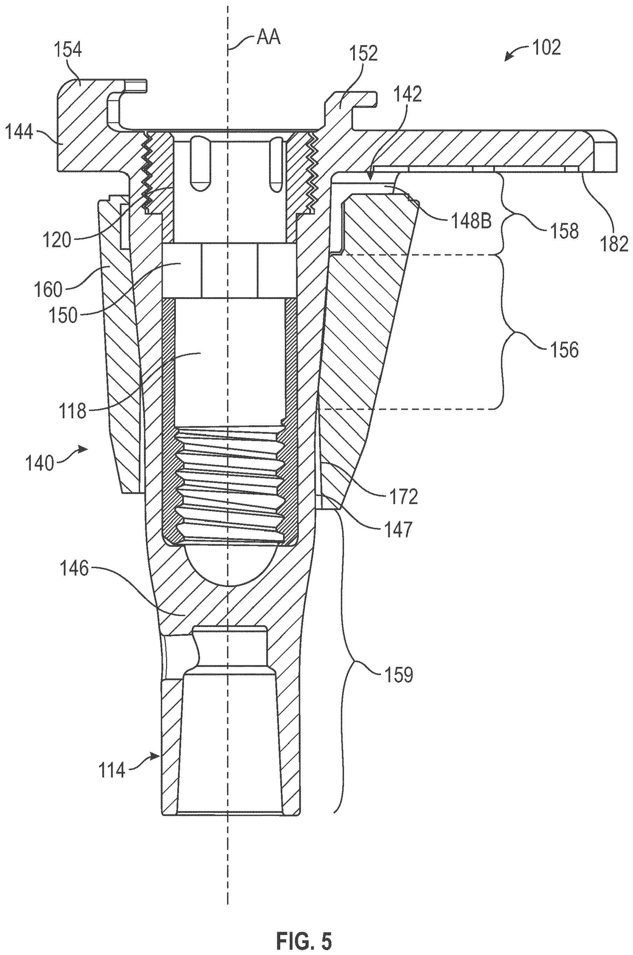

FIG. 5 is a side cross-sectional view of tibial component 102 of FIGS. 4A and 4B. Slotted sleeve 140 can be positioned over stem 146 so that internal surface 172 of slotted sleeve 140 can engage external surface 147. Internal surface 172, or a portion thereof, can be configured to engage with external surface 147, or a portion thereof, using a tapered interface, such as a Morse taper. In examples, external surface 147 of stem 146 can be configured to have a Morse taper and internal surface 172 of slotted sleeve 140 can be configured to have a mating recess such that a self-holding connection is made. Such a configuration is discussed in greater detail in U.S. Pat. No. 6,911,100 to Gibbs et al., which is hereby incorporated by reference in its entirety for all purposes. In other examples, other tapered connections can be used, such as described in U.S. Pub. No. 2015,0216667 to Monaghan, which is hereby incorporated by reference in its entirety for all purposes. In examples, sleeve 140 and stem 146 can both be fabricated of metal material, such as stainless steel or titanium, which can facilitate a Morse taper connection. In examples, sleeve 140 can be fabricated from a porous metal structure. Use of a Morse taper can allow sleeve 140 to be attached to stem 146 without the use of fasteners or additional components. For example, sleeve 140 can be spaced from lower surface 182 of tray plate 144 and the use of fasteners to connect sleeve 140 to tray plate 144 can be avoided.

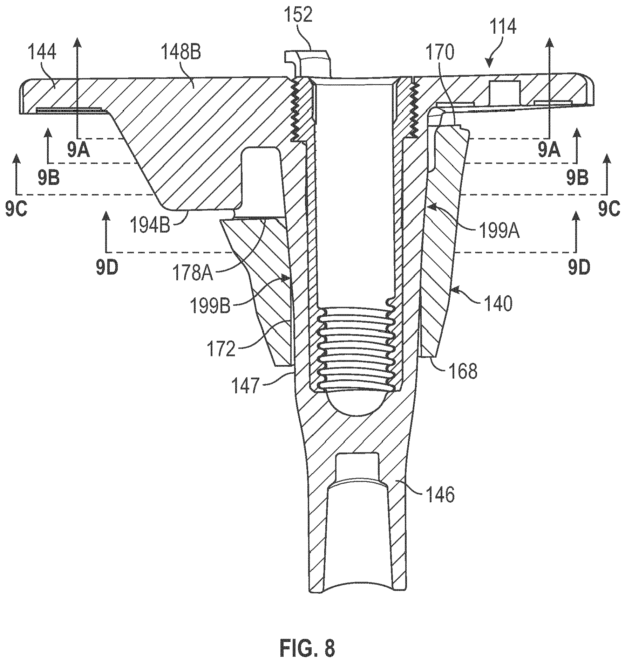

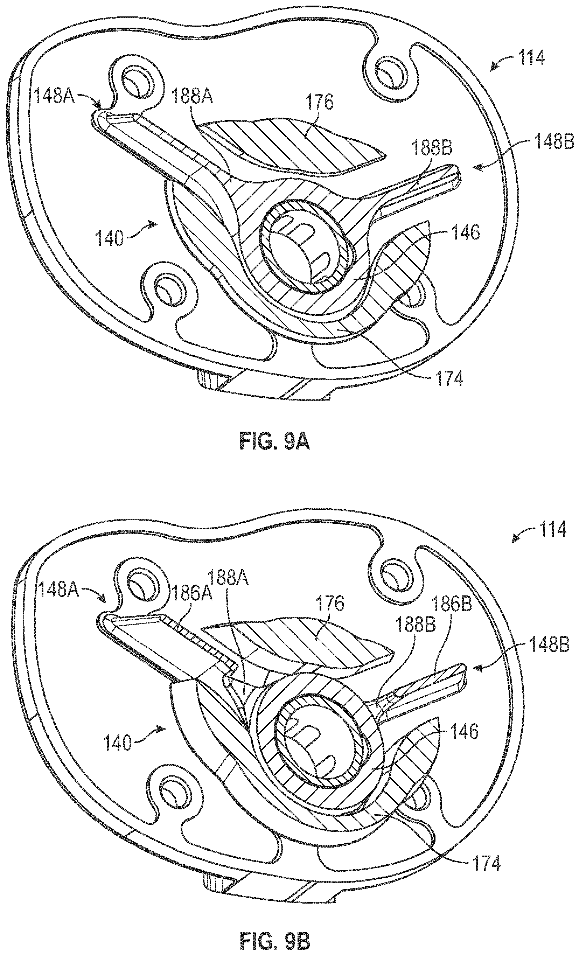

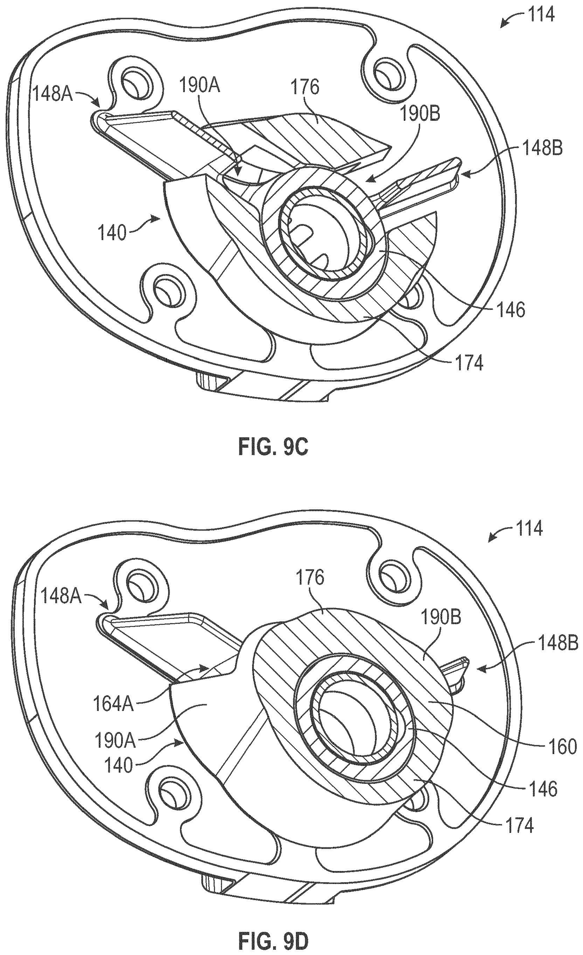

As can be seen in FIG. 5, the central portion of sleeve 140 can engage stem 146 unincumbered from segmented keel 142 in zone 156. Thus, internal surface 172 and external surface 147 can engage along a three-hundred-sixty-degree interface relative to axis AA in zone 156, as can be seen in FIG. 9D. However, a proximal portion of sleeve 140 can be positioned at the same axial position relative to axis AA as segmented keel 142. As such, only portions of internal surface 172 can engage external surface 147 in zone 158, as can be seen in FIG. 9C. Additionally, other portions of sleeve 140 can be completely unattached to stem 146, such as at the proximal-most portion, as can be seen in FIGS. 9A and 9B. Stem 146 can extend in zone 159 unengaged with sleeve 140. As will be discussed in greater detail with reference to FIGS. 8 - 9D, the proximal-most portion of sleeve 140 in zone 158 can engage stem 146 along an anterior-most segment and posterior-most segment of stem 146 due to the location of wing 148A and wing 148B.

FIG. 6A is a rear view of tibial tray 114 of FIGS. 1-5. FIG. 6B is a close-up view of tibial tray 114 of FIG. 6A showing slots 190A and 190B forming the segmented keel wings 148A and 148B, respectively, at stem 146. FIGS. 6A and 6B are discussed concurrently.

Tibial tray 114 can comprise tray plate 144 and stem 146. Tray plate 144 can comprise upper surface 180 upon which bearing component 116 can be seated. Lower surface 182 can be disposed opposite upper surface 180. Perimeter 184 can connect upper surface 180 and lower surface 182 and can have an outer perimeter shape matching that of a proximal portion of a tibia bone. Stem 146 can extend from lower surface 182 along axis AA. As discussed, stem 146 can have a proximal-most portion in zone 158 and a central portion in zone 156. The proximal-most portion can be the axial portion co-existent with segmented keel 142 and the central portion can be distal of the proximal-most portion. Stem 146 can include a distal-most portion in zone 159 that is distal of the central portion and has a smaller diameter than the central portion to facilitate positioning of sleeve 140 around stem 146.

Segmented keel 142 can additionally extend from lower surface 182. Segmented Keel 142 can be connected to tray plate 144 and stem 146. In particular, segmented keel 142 can comprise wing 148A and wing 148B that can extend radially outward from stem 146 along tray plate 144. Wing 148A can comprise distal portion 186A, proximal portion 188A and slot 190A. Wing 148B can comprise distal portion 186B, proximal portion 188B and slot 190B. Distal portion 186A can comprise angled end 192A and flat end 194A. Proximal portion 188A can comprise trough 198A and sidewall 196A. Distal portion 186B can comprise angled end 192B and flat end 194B. Proximal portion 188B can comprise trough 198B and sidewall 196B. Slot 190A can be formed by trough 198A, and slot 190B can be formed by trough 198B. As discussed below, the shapes of wings 148A and 148B and slots 190A and 190B can be configured to accommodate a machining tool for the shaping of stem 146.

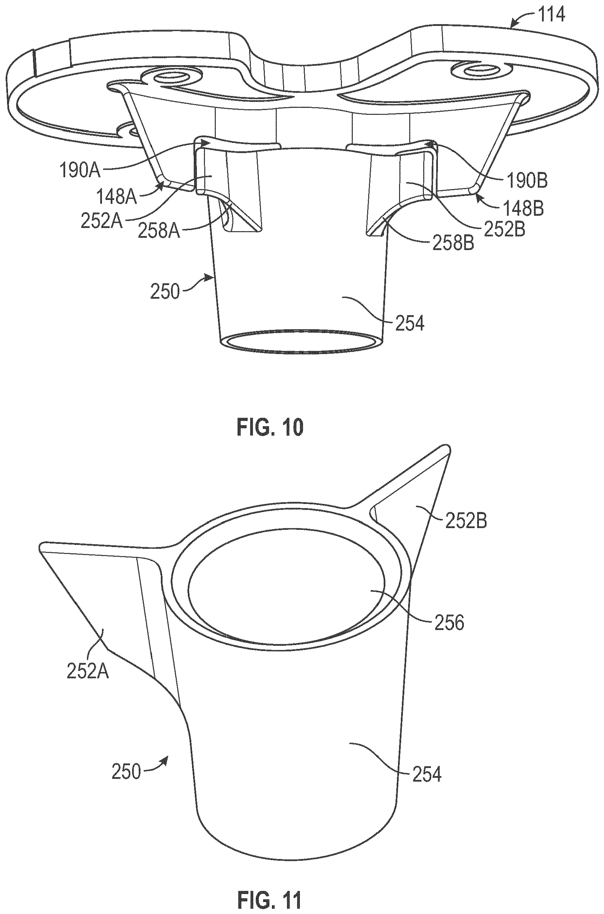

All or some of stem 146 in central zone 156 and all or some of stem 146 in proximal-most zone 158 can be formed to have a tapered surface for a Morse taper. For example, the proximal portion of stem 146 in zone 156 and the distal portion of stem 146 in proximal-most zone 158 can be machined to have a Morse tape. Machining tool 200 (shown schematically in FIG. 6A) can comprise cylindrical body 202 having an interior machining surface 204 and outer wall 206. Cylindrical body 202 can have walls 208 between interior machining surface 204 and outer wall 206. The machining process can involve positioning machining tool 200 over stem 146. Cylindrical body 202 can be reciprocated and/or rotated to engage interior machining surface 204 with external surface 147 of stem 146 to form the tapered connection surface, e.g., the Morse taper surface. In order to allow machining tool 200 to be brought into close proximity to tray plate 144, wings 148A and 148B can include slots 190A and 190B, respectively. Slots 190A and 190B can comprise notches that can accept a machining tool or that can accept portions of a sleeve. The overall outer diameter of cylindrical body 202 can be less than the distance between sidewall 196A and sidewall 196B. Additionally, the radial width of slots 190A and 190B relative to axis AA, e.g., the lengths of trough 198A and trough 198B, can be greater than the radial thickness of outer wall 206. As such, portions of sleeve 140 can be configured to engage stem 146 proximate tray plate 144 despite the presence of segmented keel 142. Furthermore, slots 190A and 190B can be used to accommodate portions of sleeve 140 in various examples. For example, portions of sleeve 140 can extend into slots 190A and 190B, as shown in FIG. 10.

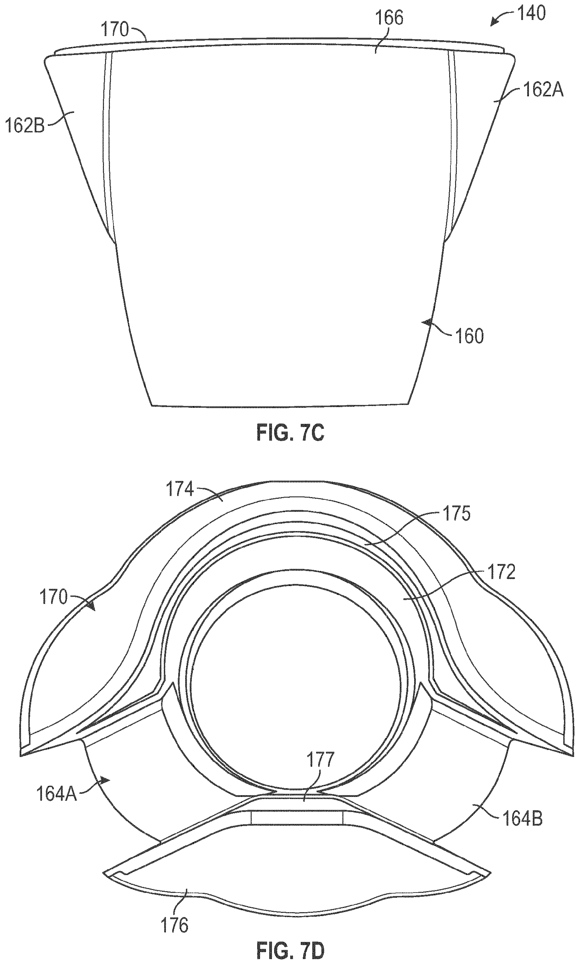

FIG. 7A is a front perspective view of slotted sleeve 140 of FIGS. 1-5 showing slots 164A and 164B extending from central passage 161. FIG. 7B is a bottom perspective view of slotted sleeve 140 of FIG. 7A showing slots 164A and 164B extending through to outer surface 166. FIG. 7C is a front view of slotted sleeve 140 of FIGS. 7A and 7B showing side lobes 162A and 162B extending from body 160. FIG. 7D is a top view of slotted sleeve 140 of FIGS. 7A - 7C showing internal ledges 175 and 177 along central passage 161. FIGS. 7A - 7D are discussed concurrently.

Slotted sleeve 140 can comprise body 160, central passage161, lobe 162A, lobe 162B, slot 164A and slot 164B. Slots 164A and 164B can comprise receptacles that can accept portions of keel wings 148A and 148B. Body 160 can comprise outer surface 166, bottom surface 168, top surface 170 and internal surface 172. Slot 164A and slot 164B can form posterior wall 174 having ledge 175 and anterior wall 176 having ledge 177 within body 160. Slot 164A can comprise bottom wall 178A, front wall 178B and back wall 178C and slot 164B can comprise bottom wall 179A, front wall 179B and back wall 179C.

FIG. 8 is cross-sectional view of tibial tray 114 of FIGS. 6A and 6B assembled with slotted sleeve 140 of FIGS. 7A - 7D taken along segmented keel wing 148B to show engagement of slotted sleeve 140 with stem 146 on anterior and posterior sides of stem 146. External surface 147 of stem 146 can engage internal surface 172 along an anterior portion at interface 199A and along a posterior portion along interface 199B. As can be seen in FIG. 8, interface 199B can be located distal of wing 148B while interface 199A can extend proximally into the axial space of wing 148B.

In examples, the axial height of distal portion 186A and distal portion 186B, e.g., the distance between tray plate 144 and flat end 194A and flat end 194B can be about one-half or less than the length of the height of sleeve 140 between top surface 170 and bottom surface 168. In additional examples, the height of distal portion 186A and distal portion 186B can be in the range of about 30% to about 60% of the height of sleeve 140.

In examples, the axial height of proximal portion 188A and proximal portion 188B, e.g., the distance between tray plate 144 and trough 198A and trough 198B can be about one-quarter or less than the length of the height of sleeve 140 between top surface 170 and bottom surface 168. In additional examples, the height of distal portions 186A and 186B can be in the range of about 0% to about 40% of the height of sleeve 140. For example, proximal portions 188A and 188B can be omitted.

In examples, top surface 170 of sleeve 140 can be configured to be below lower surface 182 of tray plate 144 when sleeve 140 is fully engaged with stem 146. In examples, bottom walls 178A and 179A can be below flat end 194A and flat end 194B when sleeve 140 is fully engaged with stem 146.

FIG. 9A, FIG. 9B, FIG. 9C and FIG. 9D are cross-sectional views of slotted sleeve 140 assembled onto tibial tray 114 showing engagement between segmented keel 142 and slotted sleeve 140 at various positions along stem 146.

FIG. 9A is taken at section 9A-9A of FIG. 8 along proximal portion 188A and proximal portion 188B and distal portion 186A and distal 186B of wings 148A and 148B. Posterior wall 174 and anterior wall 176 can be disengaged with stem 146. Additionally, posterior wall 174 and anterior wall 176 can be spaced from lower surface 182 of tray plate 144. Disengagement between sleeve 140 and tray plate 144 can avoid imparting stresses to tray plate 144 from sleeve 140.

FIG. 9B is taken at section 9B-9B of FIG. 8 along distal portion 186A and distal portion 186B of wings 148A and 148B where stem 146 and sleeve 140 are not engaged. Posterior wall 174 and anterior wall 176 can remain disengaged from stem 146. Spacing of stem 146 from sleeve 140 can be provided by ledge 175 and ledge 177. Such a spacing provides a runway to allow a Morse taper between internal surface 172 of sleeve 140 and external surface 147 of stem 146 to interlock. That is, if the tapered surfaces of external surface 147 and internal surface 172 were advanced all the way to lower surface 182 of tray plate 144, it could interfere with the ability of the Morse taper to completely engage. For example, machining tool 200 (FIG. 6A) need not advance all the way to lower surface 182 of tray plate 144. Such a spacing can be provided by proximal portions 188A and 188B to prevent advancing of machining tool 200.

FIG. 9C is taken at section 9C-9C of FIG. 8 along distal portion 186A and distal portion 186B of wings 148A and 148B where stem 146 and sleeve 140 are engaged. Posterior wall 174 and anterior wall 176 can be engaged with stem 146. Engagement of stem 146 with sleeve 140 can be provided below or distal of ledge 175 and ledge 177. As such, the anterior-most portion of sleeve 140 at anterior wall 176 can be engaged with stem 146 using a tapered connection, such as a Morse taper. Likewise, the posterior-most portion of sleeve 140 at posterior wall 174 can be engaged with stem 146 using a tapered connection, such as a Morse taper. The Morse taper surfaces shown in FIG. 9C can extend within the same axial space as wings 148A and 148B to provide additional coupling force beyond what would be provided if machining tool 200 were only advance to wings 148A and 148B and machining tool 200 did not enter slots 190A and 190B.

FIG. 9D is taken at section 9D-9D of FIG. 8 along stem 146 and sleeve 140 where sleeve 140 is engaged with stem 146 along a three-hundred-sixty-degree perimeter. Body 160 of sleeve 140 can be engaged with stem 146. Engagement of stem 146 with sleeve 140 can be provided around a complete three-hundred-sixty-degree perimeter of stem 146 using a tapered connection, such as a Morse taper. As such, sleeve 140 can be firmly engaged with stem 146 without the use of fasteners. Lobes 164A and 164B can extend radially outward from body 160 to cover slots 190A and 190B.

FIG. 10 is a bottom rear perspective view of tibial tray 114 of FIGS. 6A and 6B assembled with winged sleeve 250. FIG. 11 is a top front perspective view of winged sleeve 250 of FIG. 10 showing wing 252A and wing 252B configured to fill-in slot 190A and slot 190B, respectively, in wing 148A and wing 148B of segmented keel 142. Winged sleeve 250 can comprise cylindrical body 254 from which wings 252A and 252B extend. Inner passage 256 can extend through cylindrical body 254. FIGS. 10 and 11 are discussed concurrently.

Cylindrical body 254 can be configured similarly to body 160 of sleeve 140, but with wings 252A and 252B replacing lobes 162A and 162B. Wings 252A and 252B can have the same or similar shape as slots 190A and 190B. Wings 252A and 252B can be slightly smaller than slots 190A and 190B to facilitate insertion of wings 252A and 252B into slots 190A and 190B. Wings 252A and 252B can have the same or similar thickness as wings 148A and 148B. In examples, wings 252A and 252B can include sloped distal surfaces 258A and 258B to extend along cylindrical body 254 to facilitate insertion into bone and provide stability. For example, distal surfaces 258A and 258B can provide cutting surfaces. Wings 252A and 252B can extend level with the proximal-most surface of cylindrical body 254 to facilitate seating within slots 190A and 190B. In examples, cylindrical body 254 can be clocked to stem 146 to facilitate orientation of wings 252A and 252B with slots 190A and 190B. For example, external surface 147 of stem 146 can include a slot or a flange that can align with a flange or slot on inner passage 256.

Wings 252A and 252B can provide additional anti-rotation capabilities to tibial tray 114, e.g., prevent tibial tray 114 from rotating within bone matter of a resected tibia. In additional examples, the thickness of cylindrical body 254 can be increased and the size of wings 252A and 252B can be decreased or wings 252A and 252B can be omitted. Thus, the added thickness of cylindrical body 254 can provide additional anchoring within bone matter. Likewise, in additional examples, wings 252A and 252B can have other shapes or thicknesses to facilitate engagement with bone matter or wings 252A and 252B can be offset from wings 148A and 148B.

As discussed herein, the segmented keels and associated slotted sleeves can 1) reduce the number of assembly steps performed during a surgical procedure by providing an integrated keel; 2) reduce the number of accessory components that might be used during a surgical procedure by eliminating a separate keel accessory; and 3) facilitate coupling of sleeves to keeled or winged stems by providing keel wings having segments or slots that can accommodate a machining tool for forming tapered surfaces, such as those used for Morse tapers.

### Examples

Example 1 is a tibial implant comprising: a tibial tray component comprising: a tray plate comprising: a proximal surface; and a distal surface; a stem extending from the distal surface; a segmented keel extending from the distal surface, wherein the segmented keel includes a first wing having a first notch proximate the stem; and a first taper extending along the stem in a first axial position axially aligned with the segmented keel.

In Example 2, the subject matter of Example 1 optionally includes wherein the first notch is adjacent the stem.

In Example 3, the subject matter of any one or more of Examples 1-2 optionally include wherein the first wing has a first height from the distal surface at the first notch and a second height from the distal surface away from the first notch, wherein the second height is greater than the first height.

In Example 4, the subject matter of any one or more of Examples 1-3 optionally include a second wing extending from the distal surface spaced apart from the first wing.

In Example 5, the subject matter of any one or more of Examples 1-4 optionally include wherein the first taper extends into the first notch.

In Example 6, the subject matter of any one or more of Examples 1-5 optionally include wherein the first taper is spaced from the distal surface of the tray plate by a portion of the first wing in the first notch.

In Example 7, the subject matter of any one or more of Examples 1-6 optionally include wherein the first taper comprises a Morse taper.

In Example 8, the subject matter of any one or more of Examples 5-7 optionally include wherein the stem includes a second taper in a second axial position at a distal tip of the stem, the second taper comprising a necked-down portion of the stem.

In Example 9, the subject matter of any one or more of Examples 1-8 optionally include a sleeve couplable to the stem, the sleeve comprising a first slot to accommodate the first wing.

In Example 10, the subject matter of Example 9 optionally includes wherein the first wing fits into the first notch to allow a proximal surface of the sleeve to come into close proximity to the distal surface of the tray plate.

In Example 11, the subject matter of any one or more of Examples 9-10 optionally include wherein the sleeve further comprises a first lobe protruding from an exterior surface of the sleeve at the first slot.

In Example 12, the subject matter of Example 11 optionally includes a channel extending into the first lobe to form an extension of the first slot.

In Example 13, the subject matter of any one or more of Examples 9-12 optionally include wherein the sleeve is configured fit into the first notch spaced apart from the distal surface of the tray plate.

In Example 14, the subject matter of any one or more of Examples 1-13 optionally include a sleeve couplable to the stem, the sleeve comprising a first sleeve wing configured to be received in the first notch.

Example 15 is a sleeve for a stem of a prosthetic implant, the sleeve comprising: an annular body; an inner passage extending through the annular body, wherein the inner passage comprises a Morse taper; and a first slot extending into the annular body to intersect the inner passage and the Morse taper.

In Example 16, the subject matter of Example 15 optionally includes wherein the annular body comprises a first lobe extending from an exterior surface of the annular body aligned with the first slot, the first lobe including a first channel extending from the first slot.

In Example 17, the subject matter of Example 16 optionally includes a second slot extending into the annular body to intersect the inner passage and the Morse taper; a second lobe extending from the exterior surface of the annular body aligned with the second slot; and a second channel extending from the second slot.

In Example 18, the subject matter of any one or more of Examples 15-17 optionally include wherein the inner passage comprises a first ledge spacing the Morse taper form a proximal surface of the annular body.

Example 19 is a prosthetic implant comprising: a prosthetic component comprising: an articulating component; a stem extending from the articulating component; and a first segmented keel wing extending form the stem; and a slotted sleeve comprising: an annular body; an inner passage extending through the annular body configured to receive the stem; and a first slot extending into the annular body to intersect the inner passage, the first slot configured to align with the first segmented keel wing.

In Example 20, the subject matter of Example 19 optionally includes wherein the first slot is radially longer than a first notch extending into the first segmented keel wing.

In Example 21, the subject matter of any one or more of Examples 19-20 optionally include wherein a proximal surface of the slotted sleeve is spaced from the articulating component when the inner passage is fully seated on the stem.

In Example 22, the subject matter of any one or more of Examples 19-21 optionally include wherein the stem and the inner passage are configured to mate using a Morse taper having axial overlap with first segmented keel wing.

Example 23 is a method of manufacturing a stemmed prosthetic component, the method comprising: fabricating the stemmed prosthetic component from a metallic material; positioning a machining tool around a stem extending from the stemmed prosthetic component; advancing the machining tool into a notch in a keel wing extending from the stem; and forming a Morse taper on the stem with the machining tool in the notch.

In Example 24, the subject matter of Example 23 optionally includes wherein the machining tool is advanced proximally past a distal-most end of the keel wing.

Each of these non-limiting examples can stand on its own, or can be combined in various permutations or combinations with one or more of the other examples.

### Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A tibial implant comprising:
a tibial tray component comprising:
a tray plate comprising:
a proximal surface; and
a distal surface;
a stem extending from the distal surface;
a segmented keel extending from the distal surface, wherein the segmented keel includes a first wing having a first notch proximate the stem; and
a first taper extending along the stem in a first axial position axially aligned with the segmented keel.

2. The tibial implant of claim 1, wherein the first notch is adjacent the stem.

3. The tibial implant of any of claims 1-2, wherein the first wing has a first height from the distal surface at the first notch and a second height from the distal surface away from the first notch, wherein the second height is greater than the first height.

4. The tibial implant of any of claims 1-3, further comprising a second wing extending from the distal surface spaced apart from the first wing.

5. The tibial implant of any of claims 1-4, wherein the first taper extends into the first notch;
optionally wherein the stem includes a second taper in a second axial position at a distal tip of the stem, the second taper comprising a necked-down portion of the stem.

6. The tibial implant of any of claims 1-5, wherein the first taper is spaced from the distal surface of the tray plate by a portion of the first wing in the first notch.

7. The tibial implant of any of claims 1-6, wherein the first taper comprises a Morse taper.

8. The tibial implant of any of claims 1-7, further comprising a sleeve couplable to the stem, the sleeve comprising a first slot to accommodate the first wing.

9. The tibial implant of claim 8, wherein the first wing fits into the first notch to allow a proximal surface of the sleeve to come into close proximity to the distal surface of the tray plate.

10. The tibial implant of any of claims 8-9, wherein the sleeve further comprises a first lobe protruding from an exterior surface of the sleeve at the first slot;
optionally further comprising a channel extending into the first lobe to form an extension of the first slot.

11. The tibial implant of any of claims 8-10, wherein the sleeve is configured fit into the first notch spaced apart from the distal surface of the tray plate.

12. The tibial implant of any of claims 1-7, further comprising a sleeve couplable to the stem, the sleeve comprising a first sleeve wing configured to be received in the first notch.

13. The tibial implant of any of claims 1-7, further comprising a sleeve couplable to the stem, the sleeve comprising:
an annular body;
an inner passage extending through the annular body, wherein the inner passage comprises a Morse taper; and
a first slot extending into the annular body to intersect the inner passage and the Morse taper,
optionally wherein the annular body comprises a first lobe extending from an exterior surface of the annular body aligned with the first slot, the first lobe including a first channel extending from the first slot;
further optionally where the sleeve further comprises:
a second slot extending into the annular body to intersect the inner passage and
the Morse taper;
a second lobe extending from the exterior surface of the annular body aligned with the second slot; and
a second channel extending from the second slot;
further optionally wherein the inner passage comprises a first ledge spacing the Morse taper form a proximal surface of the annular body.

14. A method of manufacturing a stemmed prosthetic component, the method comprising:
fabricating the stemmed prosthetic component from a metallic material;
positioning a machining tool around a stem extending from the stemmed prosthetic component;
advancing the machining tool into a notch in a keel wing extending from the stem; and
forming a Morse taper on the stem with the machining tool in the notch.

15. The method of claim 14, wherein the machining tool is advanced proximally past a distal-most end of the keel wing.
